# EUROPEAN PATENT APPLICATION

(11) **EP 1 245 670 A2**
(43) Date of publication of application: **02.10.2002**
(21) Application number: 02252234.6
(22) Date of filing: 27.03.2002
(51) Int. Cl.: C12M 3/04

(54) **Method and apparatus for culturing cells**

(30) Priority: 27.03.2001 US 278955 P
(71) Applicant: Becton, Dickson and Company, Franklin Lakes, NJ 07417-1880 (US)
(72) Inventor: Liebmann-Vinson, Andrea, Willow Springs, NC 27592 (US); Meyer, Mary, Durham, NC 27705 (US); Hsieh, Helen V., Durham, NC 27713 (US)
(74) Representative: McCarthy, Denis Alexis

(57) **Abstract**

A method and apparatus for improving the efficiency of the growth of cell and tissue cultures *in vitro* is disclosed. The improvement in efficiency is achieved through the use of a scaffold formed from an open cell polymer foam that has been surface treated by an oxidative plasma discharge. In one embodiment, the polymer foam is a polystyrene foam treated with an oxygen gas plasma to functionalize the surface of the polymer. In the preferred embodiment, the scaffold is used as an insert for a bioreactor to culture cells and tissue. The scaffold is formed from a porous polymer structure having a continuous polymer matrix with a plurality of interconnected open pores. The pore size typically ranges from about 50 microns to about 500 microns.

## Description

This application claims the benefit of U.S. Provisional Application Serial No. 60/278,955, filed March 27, 2001, the content of which is expressly incorporated herein by reference.

### FIELD OF THE INVENTION

The present invention is directed to a method and apparatus for culturing cells and tissue. More particularly, the invention is directed to a synthetic porous extracellular matrix, also commonly called a scaffold, for *in vitro* three-dimensional tissue or cell culture having a treated surface conducive to cell attachment and growth.

### BACKGROUND OF THE INVENTION

Commonly, human and animal cell cultures *in vitro* are performed in monolayers on an artificial substrate. Culture vessels, such as dishes, flasks and multiwell plates provide the planar two-dimensional surfaces upon which the cell monolayer is formed. Polystyrene is a commonly used material for the production of such culture vessels. Oxidative plasma treatment processes for treating polystyrene surface rendering them conducive for cell attachment are also known. Such treatments are critical for the culture of anchorage dependent cells, which must first attach and spread before they undergo further cell division. The effectiveness of culture substrates to support cell attachment depends on various factors including the available surface area, surface chemical composition and surface wettability. For many cells, the culture yield depends on the ability of the cells to anchor to the surface of the culture vessel.

Numerous processes and methods are known in the art for culturing anchorage dependent human and animal cells. The final application of cells and/or their products determine which method is most suitable for use.

Many standard methods for tissue and cell culture are limited by the need of careful supervision and control of the growth media that provides nutrients to the tissues and cells. The control of the supply of nutrients limits the amount of cells and tissue that can be cultured at one time.

Tissue engineering often uses extracellular matrices to support the growth of tissue cells. The tissue cells are transplanted onto a macroporous matrix to create new tissues that are structurally integrated with the host tissue. The resulting tissues are potentially capable of replacing all function of lost or damaged tissue in the patient. The tissue cells are typically cultured on the synthetic matrix *in vitro* and then subsequently implanted *in vivo* where the synthetic matrix guides new tissue formation.

Synthetic extracellular matrices are required to facilitate controlled delivery or localization of cells, and maintain a three-dimensional space for tissue formation. The matrices are also used to guide gene expression and tissue development in order to yield function tissues. Naturally occurring matrices are often made from fibrous proteins, such as collagen and elastin that are able to form highly organized structures and impart strength and stability to the natural tissue. Other materials including glycosaminoglycans containing polysaccharide chains that form highly hydrated gels. The polysaccharide components resist compressive forces on the matrix while the fibrous proteins provide tensile strength.

Synthetic polymers have also been used to form structural scaffolds to provide strength and structural integrity to engineered tissues. The polymers can be biodegradable polymers, such as polyglycolic acid, in the form of fiber or sponge-based scaffolds.

Various devices have been proposed to improve the growth of cells and tissues. One example is disclosed in U.S. Patent No. 5,763,267 to Kurjan et al. The apparatus disclosed therein relates to a large scale culturing and packaging of cell suspensions. The apparatus includes a plurality of tissue scaffolds with a treatment chamber. A fluid inlet, a fluid outlet, and a fluid reservoir are included to circulate the nutrient media through the apparatus.

Another method for culturing tissue and cells is disclosed in U.S. Patent No. 5,266,480 to Naughton. This method provides a three-dimensional matrix and seeds the matrix with the desired cells. The culture is maintained to produce three-dimensional tissues that can be used for various medical uses.

Another device commonly used for culturing tissues and cells is the roller bottle as disclosed in U.S. Patent No. 5,010,013 to Serkes et al. This roller bottle has a plurality of corrugations to increase the surface area of the bottle for supporting the cells. Many types of cells grow slowly and require a support or substrate for the cells to attach themselves. This roller bottle is an example of a device intended to increase the surface area for supporting the cells.

The prior apparatus and methods have been generally effective for their intended purpose. However, there is a continuing need in the industry for improved methods and devices for culturing cells and tissue.

Citation of the foregoing patent documents is not intended as an admission that any of the foregoing are pertinent prior art. All statements as to the date or representation as to the contents of these documents is based on the information available to the inventors and does not constitute any admission as to the correctness of the dates or contents of these documents.

### SUMMARY OF THE INVENTION

The present invention is directed to a method and apparatus for culturing cells and tissues. More particularly, the invention is directed to a method and apparatus for growing cells and tissues *in vitro* on a porous matrix having one or more internal surfaces including pore internal surfaces that are treated to promote cell growth. Accordingly, a primary object of the invention is to provide a method and apparatus for improving the efficiency of the growth of cell and tissue cultures *in vitro.*

Another aspect of the invention is to provide a porous cell and tissue scaffold having a surface area to promote the growth of cells and tissue.

A further aspect of the invention is to provide an open cell polystyrene matrix suitable as a scaffold to support the cell and tissue cultures and promote cell growth.

Another object of the invention is to provide an open cell polystyrene scaffold that has been treated to functionalize the surfaces to effectively anchor cells and tissue during culturing of the cells or tissue.

Still another aspect of the invention is to provide a method for culturing cells and tissues using a scaffold made from an open cell polystyrene that has been treated by oxidative radio frequency etching to enhance the anchoring of cells and tissues during cell and tissue growth.

A further aspect of the invention is to provide a three-dimensional scaffold for cell and tissue culture where the scaffold is a porous open cell polystyrene foam having a pore size of about 50 microns to about 200 microns.

Still another aspect of the invention is to provide a scaffold for use as an insert in a bioreactor where the scaffold is a porous open cell foam having a surface area that is functionalized to support the growth of cells and tissue.

The aspects and advantages of the invention are basically attained by providing a vessel and a three-dimensional scaffold support positioned in the vessel for supporting the growth of cells. The support comprising a porous, open pore polymer foam member. The polymer foam member has a substantially continuous polymer matrix with a plurality of pores defining internal surfaces. The polymer matrix is subjected to oxidative radio frequency treatment to functionalize the internal surfaces and promote attachment of cells to surfaces of the internal surfaces of the open pores.

The aspects and advantages of the invention are further attained by providing a method of culturing cells which comprises the steps of providing a three-dimensional scaffold support formed from an open pore polymer foam member which has a substantially continuous polymer matrix with a plurality of open pores defining internal surfaces, where the polymer matrix has been subjected to oxidative radio frequency etching, and seeding the scaffold with cells supplying a nutrient medium to the cell and culturing the cells *in vitro* on the scaffold.

The aspects and advantages of the invention are also attained by providing a method for preparing a porous polymer scaffold for supporting the growth of cells. The method comprises the steps of providing a three-dimensional member formed from an open pore polymer matrix having a plurality of interconnected pores defining internal surfaces of the member, and etching the internal surfaces of the member by oxidative radio frequency etching to oxidize surfaces of the member.

These aspects, advantages and other salient features of the invention will become apparent from the annexed drawings and the following detailed description of the invention.

### BRIEF DESCRIPTION OF THE DRAWINGS

The following is a brief description of the drawings, in which:
Figure 1 is a perspective view of the roller bottle-type reaction container in one embodiment of the invention; and
Figure 2 is a cross-sectional view of the container of Figure 1 showing the porous scaffold.

### DETAILED DESCRIPTION OF THE INVENTION

The present invention is directed to a method and apparatus for culturing cells. More particularly, the invention is directed to a method and apparatus for culturing cells and tissues *in vitro* using a porous scaffold.

The scaffold of the invention is a porous three-dimensional member formed from an open cell polymer foam. The porous foam scaffold has a substantially continuous polymer phase with highly interconnected pores. The pores form substantially continuous channels through the polymer matrix. The channels are connected to form a substantially continuous network of channels in the polymer phase with a very high surface area suitable for cell growth. Preferably, the channels or pores are distributed throughout the polymer phase to promote diffusion of cells and cell nutrients throughout the pores.

The scaffold of the invention is suitable for culturing various cells and tissues. The scaffold is particularly suitable for *in vitro* cell and tissue culture. Examples of cells that can be cultured include tumor cells, bone marrow cells, skin cells, liver cells, pancreas cells, kidney cells, neurological tissue cells, adrenal gland cells, and the like.

The polymer scaffold of the present invention is suitable for implants for *in vivo* cell and tissue growth and particularly for *in vitro* cell growth to support cells and tissues in various bioreactors. The bioreactors can be, for example, culture dishes, flasks, bottles, or roller bottles. The scaffold according to the invention is preferably an insert for use in a bioreactor that can be inserted and removed as desired. Alternatively, the bioreactor itself can be produced in accordance with the invention to promote the growth of cells and tissues directly on the surfaces of the bioreactor.

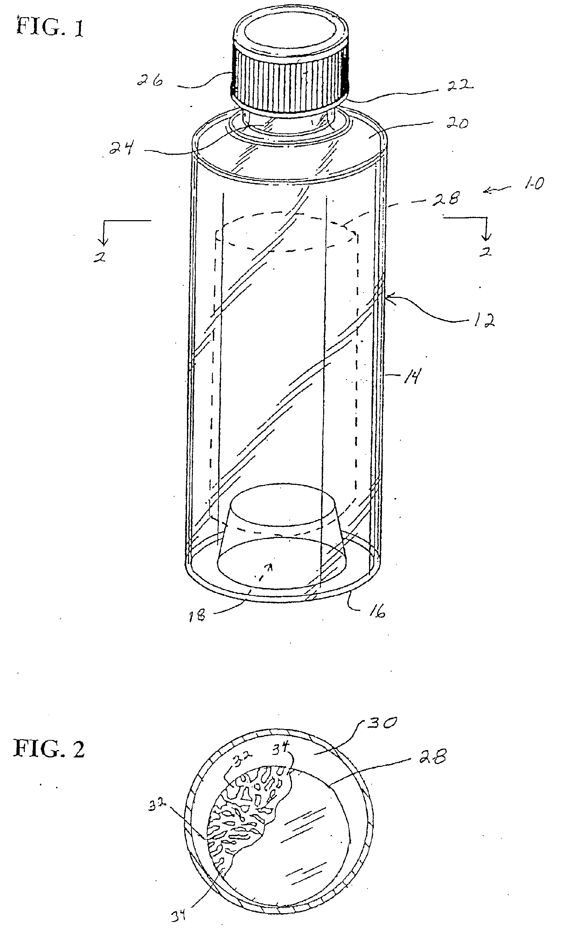

In one embodiment of the invention shown in Figures 1 and 2, a bioreactor 10 is in the form of a roller bottle 12. It will be appreciated that roller bottle 12 is intended to illustrate the various aspects of the invention and that the bioreactor can have other shapes, sizes and forms. In the embodiment illustrated, roller bottle 12 has a substantially cylindrical side wall 14 and a closed bottom end 16. In the illustrated embodiment, bottom end 16 includes a recessed portion 18 to increase the internal surface area of bottle 12. Roller bottle 12 also includes a top wall 20 having an opening formed by a neck 24 extending from top wall 20. A closure member 26 is provided to fit on neck 24 to close bottle 12 during use. Preferably, closure member 26 forms a leakproof seal to contain a liquid sample during use. In embodiments of the invention, closure member 26 can have an air permeable membrane as known in the art.

Roller bottle 12 includes a porous scaffold 28 disposed within a cavity 30. In the embodiment illustrated, scaffold 28 has a substantially cylindrical shape and an overall dimension slightly less than the internal dimensions of cavity 30. In this embodiment, scaffold 28 is provided as an insert that is dimensioned to move within cavity 30 to continuously contact a cell growth supporting medium as bottle 12 is rotated during the cell growing process. Preferably, scaffold 28 has a shape and dimension to avoid excessive impact with the inner surface of roller bottle 12 to reduce the occurrence of cells being dislodged from the surface of scaffold 28. As shown in Figure 2, scaffold 28 includes a substantially continuous polymer matrix 32 having interconnected pores 34.

In further embodiments, scaffold 28 is mounted within roller bottle 12 or other bioreactor in a fixed position. In one embodiment, scaffold 28 is removable from roller bottle 12. In the illustrated embodiment, scaffold 28 is larger than opening 22 of roller bottle 12 and is not intended to be removed. In embodiments where the scaffold is removable, the bioreactor is appropriately dimensioned to allow easy separation of the scaffold from the bioreactor. One example of a roller bottle having removable end caps so that a support surface can be inserted is disclosed in U.S. Patent No. 4,912,058 to Mussi et al., which is hereby incorporated by reference in its entirety.

The method of the invention for culturing cells and tissue provides a culture vessel such as roller bottle 12 of the embodiment of Figures 1 and 2 having porous scaffold 28 enclosed therein. Porous scaffold 28 is seeded with the target cells and supplied with a nutrient growth medium capable of supporting the growth of the target cells. Roller bottle 12 is then placed in a suitable rolling device as known in the art under appropriate temperature conditions and for a time sufficient to culture the target cells on scaffold 28.

The illustrated embodiment relates to a roller bottle for culturing cells. The scaffold of the invention can also be used for other bioreactors capable of supporting and promoting the growth of cells and tissue. Examples of suitable bioreactor culturing vessels such as the vessel disclosed in U.S. Patent No. 5,391,496 to Kayal et al., which is hereby incorporated by reference in its entirety. In further embodiments, the bioreactor can have a plurality of chambers capable of supporting a culture medium and a cell or tissue scaffold. An example of a suitable bioreactor is disclosed in U.S. Patent No. 5,763,267 to Kurjan et al., which is hereby incorporated by reference in its entirety.

The scaffold of the invention can be made from various polymers that are amenable to surface oxidation or functionalization by a suitable treatment step. Subjecting the surface of the porous polymer matrix used to form the scaffold to an oxidizing process has been found to produce functional groups on the surface of the polymer. In particular, it has been found that a plasma oxidation is able to modify and functionalize the polymer throughout the polymer matrix. The functionalization promotes the attachment of cells to the surfaces of the polymer during culturing while allowing the cells to be removed and recovered by various recovery techniques.

In a preferred embodiment of the invention, scaffold 28 is made from a porous open cell polymer, and particularly a polystyrene foam that is subjected to an oxidative radio frequency etching. It has been found that oxidative radio frequency etching of an open cell polystyrene scaffold sterilizes the polystyrene and functionalizes the surfaces of the polystyrene throughout the polymer matrix. The functionalization of the polystyrene produces a surface that is able to promote the anchoring of cells and the culturing of the cells. The radio frequency etch is able to penetrate effectively the pores of the open cell polystyrene to expose the internal surfaces of the pores to the plasma to oxidize and functionalize the surfaces of the pores.

In a preferred embodiment, the scaffold is a three-dimensional structure having a length, width and thickness made from a porous open cell polystyrene. The open cell polystyrene preferably has a porosity of about 70% to about 75% and an active surface area of at least about 10 m²/g. Preferably, the polymer foam has a porosity of at least 90%, and more preferably of at least about 93%. The surface area of the polymer matrix is about 10 m²/g to about 50 m²/g. The open pores of the polystyrene foam are preferably interconnected to form substantially continuous channels through a continuous polymeric matrix. The channels are preferably interconnected to form a network of channels that enable nutrient medium to pass through the channels to promote efficient cell growth.

The dimensions and particularly the diameter of the pores can vary depending on the cells or tissue being cultured. The dimensions of the pores must be sufficiently large to allow cells to enter the pores and to allow the exchange of nutrients to the cells through the pores. In embodiments of the invention, the pore size can range from about 50 microns to about 500 microns, and preferably about 50 microns to about 200 :microns. In one embodiment, the pores have an average diameter of about 90 microns to about 100 microns. The pores are preferably of sufficient size to support the cells during growth, while allowing an aqueous nutrient medium to flow through the pores and to enable the pores to release the resulting cell growth by rinsing or other cell recovery methods. The pore size can vary depending on the process used to produce the scaffold. In certain embodiments, the pores are substantially two different sizes that are interconnected to form substantially continuous channels. In other embodiments, the scaffold is formed from a continuous polymer matrix having substantially uniform size pores that are interconnected to form substantially continuous channels.

In the illustrated embodiment, the scaffold of the invention is a cylindrical block-like member. In further embodiments, the scaffold can have any desired shape suitable for use in the bioreactor. In one example, the scaffold has a disk-like shape that can be placed in a culture dish or other suitable bioreactor. The scaffold can be in the form of various cell culture dishes, tissue culture dishes and multi-well plates. The open pore foam of the scaffold is particularly desirable in that the structure allows for easy rinsing and detachment of cells using various known cell recovery techniques and materials. One suitable mechanism for rinsing and detaching cells from the scaffold uses a solution containing a proteolytic enzyme, such as trypsin. Other suitable mechanisms for detaching cells include sonication or agitation so long as the force applied to the cells does not induce lysis. However, if the cells are ultimately used in nucleic acid or protein extraction assays (e.g.,to isolate cell products, metabolites, or cell membrane surface molecules or moieties) prevention of lysis is less important. The skilled artisan will appreciate that any method known in the art is useful in rinsing or detaching the cultured cells from the scaffold as befits ultimate use of the cells from the culture.

The scaffold of the invention has an advantage over conventional beads in cell and tissue culture devices in that the scaffold is not consistently impacted against the wall of a vessel or other beads during the culturing process. The conventional beads support the cells only or at least primarily on the surface and result in cells being dislodged from the surface as the beads contact the wall of the bioreactor and the other beads. In addition, the recovery of the scaffold from the nutrient growth medium is simplified by the scaffold compared to the recovery of plastic beads. This generally results in lower cell losses and higher culture yields. In addition, the three-dimensional scaffolds may maintain the phenotype of certain cell lines in contrast to conventional flat surfaces that typically cause cell lines to change their phenotype.

In preferred embodiments of the invention, scaffold 28 is an insert that can be placed in a suitable bioreactor. The removable scaffold can be a single cube, block, sphere, tube, rod, disc, membrane, film, sheets, or other structure of an appropriate shape and size of the bioreactor. In further embodiments, the scaffold can be formed from fibers that are formed into a bundle. In further embodiments, the fibers can be a woven or non-woven mat. The fibers are also formed from a polymer matrix having an open pore structure with continuous channels. The surfaces of the fibers are subject to oxidative plasma etching to functionalize the surfaces of the fibers and the polymer matrix.

The scaffold of the invention can be made by various processes as known in the art for producing open pore polymeric foams. In a preferred embodiment, the scaffold is made from a porous polystyrene body. The polystyrene is preferably a high molecular weight, rigid polymer. In further embodiments, the polystyrene can be a flexible polymer. Other suitable polymers can also be used that are non-reactive with the growth medium and do not interfere with the culturing of the cells or tissue. In other embodiments, the polymer can be a bioerodible polymer. Examples of suitable biodegradable or bioerodible polymers include polyglycolic acid, polylactic acid, polyethylene oxide/ polypropylene terephthalate, polycaprolactones, polyhydroxybutyrates and copolymers of polyesters, polycarbonates, polyanhydrides and poly(orthoesters). Other suitable polymers include polyamino carbonates, polyacrylates and polyurethanes.

The scaffolds can be made by various processes that are able to produce an open pore foam structure. The foam can be produced, for example, by suitable gaseous expanding or blowing agents. The porous polymer matrix produced by the gas foaming method uses a solid polymer member that is exposed to high pressure carbon dioxide to saturate the polymer. The release of the pressure results in nucleation and expansion of the dissolved carbon dioxide to form macropores of about 100 microns and a porosity of 93%. Gas foaming has the advantage of avoiding the use of solvents that can remain and interfere with cell and tissue growth. This method is limited to polymers that are able to absorb carbon dioxide under pressure.

In one embodiment, the scaffold is made by a solvent casting, particulate leaching process. In this process, an amount of the selected polymer is dissolved in a suitable solvent or mixture of solvents. A second solvent can be added to result in a phase separation on cooling of the dissolved polymer. The solution of the polymer generally contains about 0.5 wt% to about 25 wt%, and typically about 10 wt% to about 20 wt% of the polymer.

An amount of particles for forming the pores in the scaffold is combined with the resulting polymer solution. The particles are selected according to the desired pore size of the scaffold. The particles can be of a single size or blend of particle sizes to provide a selected distribution of pores sizes in the polymer matrix. The particles are non-toxic, biocompatible substances that are insoluble or substantially insoluble in the organic solvent used to form the polymer solution. In preferred embodiments, the particles are readily water soluble. Examples of suitable particles include crystalline substances, such as biologically acceptable alkali metal salts and alkaline earth metal salts. The salts are typically halides, phosphates and sulfates. In alternative embodiments, the particles can be crystals of sugars or microspheres of water soluble polymers or proteins, such as albumin. Sodium chloride is typically used since it is readily available, water soluble and non-toxic.

The scaffold is produced by forming a bed of the particles in a suitable container or mold and pouring the heated polymer solution over the particles. Alternatively, the particles can be mixed with the polymer solution and poured into a mold. After the polymer solution is completely mixed with the particles, the solution is cooled to solidify the polymer. The organic solvent is then removed under a vacuum to form the polymer matrix. The particles are then leached from the polymer matrix using a suitable solvent for the particles. The particles are dissolved to produce the open-cell foam scaffold.

In a phase separation process, a polymer is dissolved in a solvent at a low temperature. A phase separation is induced by lowering the temperature and quenching to produce a two-phase solid. The solidified solvent is removed by sublimation to produce the polymer matrix.

After the polymer matrix is created, the polymer is subjected to an oxidative treatment to form functional groups on the surfaces of the scaffold. The oxidative treatment is applied to penetrate the pores and functionalize the polymer surface in the polymer matrix. The oxidative treatment typically diffuses through the pores of the polymer matrix to the interior and interstitial surfaces. The oxidative treatment results in modifying the surfaces to replace hydrogen atoms of the polymer with oxygen or nitrogen atoms to form hydroxyl, amino, carbonyl and carboxyl groups.

In a preferred embodiment, the scaffold is subjected to an oxidative treatment such as a radio frequency oxidative plasma treatment. The treating atmosphere contains reactive gas phase atomic oxygen and radicals that contact the polymer surface to produce functional groups. The atomic oxygen and radicals are generated from a gas source in a non-equilibrium, low pressure environment and delivered to the polymer surface by a convective and diffusion transport. The radicals are generated from a gas source by exposing the gas to high energy conditions to produce a gas plasma discharge. The discharge can be produced by radio frequency, microwave, corona discharge or direct current discharge as known in the art. The discharge can also be produced from laser photolysis, high-powered UV/VUV lamp driven photolysis, high energy electron beams, microwaves, direct current power supplies, and other high intensity ionizing or radical forming radiation sources. Plasma gas discharge is one preferred method for treating the polymers of the present invention. Suitable methods and devices for treating the internal surfaces of the polymeric foam are disclosed in U.S. Patent Nos. 5,141,806 and 5,215,790 to Koontz, which are hereby incorporated by references in their entirety.

The gas source for the plasma discharge is preferably oxygen. In further embodiments, the gas source can be ammonia or mixtures of nitrogen and hydrogen. The gas source can be used in substantially pure form or diluted with gas such as helium or argon. Diluted gases can be advantageous in producing longer lasting and higher concentration reactive radicals in the reactor. An example of a gas plasma reactor is disclosed in U.S. Patent No. 5,332,551 to Koontz, which is hereby incorporated by reference in its entirety. In preferred embodiments, the scaffold is a polystyrene foam subjected to oxidative radio frequency treatment containing reactive atomic oxygen.

In further embodiments, the surfaces of the polymer can be coated or treated with a suitable material attached to the selected components. For example, prior to oxidative treatment, the polymer surfaces can be coated with a polyether, such as polyethylene oxide. Upon oxidation, the resulting functionalized polyethylene oxide coating is able to attach to peptides, proteins and other biomolecules.

The references cited above are all incorporated by reference herein, whether specifically incorporated or not.

Having now fully described this invention, it will be appreciated by those skilled in the art that the same can be performed within a wide range of equivalent parameters, concentrations, and conditions without departing from the spirit and scope of the invention and without undue experimentation.

## Claims

1. A porous three-dimensional scaffold suitable for cell or tissue growth, said scaffold having multiple surfaces comprising an open cell polymer matrix having pores and channels that form a substantially continuous network of channels which promote the diffusion of cells and cell nutrients, wherein the surfaces of said scaffold and network including the internal pore surfaces have been functionalized to promote cell attachment.

2. The scaffold of claim 1 wherein the matrix has a porosity of at least 70%.

3. The scaffold of claim 1 or 2 wherein the functionalization is obtainable by an oxidative plasma treatment.

4. The scaffold of any one of claims 1 to 3 wherein the average diameter of the pores is 50 microns to 500 microns.

5. The scaffold of any one of the preceding claims having a polyether coating.

6. A method of culturing cells or tissues comprising:
seeding the three-dimensional scaffold of any one of the preceding claims with a cell or tissue, and
culturing the seeded cell or tissue.

7. The method of claim 6 further comprising recovering the scaffold after the culturing step.

8. A cell or tissue culture apparatus comprising:
a bioreactor, and
the three-dimensional scaffold as claimed in any one of claims 1 to 6.

9. The apparatus of claim 8 wherein the bioreactor is a culture dish, flask, bottle, or roller bottle.

10. The apparatus of claim 9 wherein the scaffold is disposed within a cavity.
